# EUROPEAN PATENT APPLICATION

(11) **EP 3 943 053 A1**
(43) Date of publication of application: **26.01.2022**
(21) Application number: 21188915.9
(22) Date of filing: 21.02.2018
(51) Int. Cl.: A61F 13/00, A61M 1/00, A61L 15/42, A61L 15/56

(54) **DIFFERENTIAL CONTRAST WOUND TISSUE INTERFACE**

(30) Priority: 21.02.2017 US 201762461603 P
(62) Divisional of application: 18708843.0
(71) Applicant: 3M Innovative Properties Co., St. Paul, MN 55133-3427 (US)
(72) Inventor: LOCKE, Christopher Brian, Bournemouth (GB); ROBINSON, Timothy Mark, Shillingstone (GB)
(74) Representative: Simmons & Simmons

(57) **Abstract**

A tissue interface having visually contrasting portions, systems for using the same, and methods of manufacturing the same are provided. The wound filler includes a first portion having a first color and a second portion having a second color. The second color contrasts to the first color. The first color and the second color both contrast to red and purple. The first color and the second color are complementary.

## Description

### RELATED APPLICATIONS

The present invention claims the benefit, under 35 USC § 119(e), of the filing of U.S. Provisional Patent Application serial number 62/461,603, entitled "DIFFERENTIAL CONTRAST WOUND FILLER," filed February 21, 2017. This provisional application is incorporated herein by reference by all purposes.

### TECHNICAL FIELD

The invention set forth in the appended claims relates generally to tissue treatment systems and more particularly, but without limitation, to a tissue interface having visually contrasting portions for identification of the tissue interface during removal from a tissue site.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

There is also widespread acceptance that cleansing a tissue site can be highly beneficial for new tissue growth. For example, a wound can be washed out with a stream of liquid solution, or a cavity can be washed out using a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed.

While the clinical benefits of negative-pressure therapy and/or instillation therapy are widely known, improvements to therapy systems, components, and processes may benefit healthcare providers and patients.

### BRIEF SUMMARY

New and useful systems, apparatuses, and methods for identifying a tissue interface in a tissue site during dressing changes and at the conclusion of therapy are set forth in the appended claims. Illustrative embodiments are also provided to enable a person skilled in the art to make and use the claimed subject matter.

For example, in some embodiments, a wound filler is described. The wound filler can include a first portion having a first color and a second portion having a second color. The second color can contrast to the first color. The first color and the second color can contrast to red and purple.

More generally, a method of manufacturing a tissue interface is provided. A first part having a first hue can be provided. A second part having a second hue can also be provided. The first hue and the second hue are distinguishable from each other and a tissue site. The first part can be joined to the second part to form a whole having visually distinguishable sections.

Alternatively, other example embodiments may describe a tissue interface for use with a negative-pressure therapy system. The tissue interface can include a first portion having a first color formed in a first pattern and a second portion having a second color formed in a second pattern. The second color can have a contrast to the first color, and the first color and the second color can have a contrast to tissue.

A system for treating a tissue site with negative pressure is also described herein. The system can include a manifold configured to be positioned adjacent to the tissue site. The manifold can have a first portion having a first color and a second portion having a second color. The second color can contrast to the first color. The first color and the second color can contrast to red and purple. The system can further include a cover configured to be positioned over the manifold to form a sealed space containing the manifold. A negative-pressure source can be fluidly coupled to the sealed space to draw fluid across the manifold, thereby generating a negative pressure.

Another tissue interface is also described herein. The tissue interface can have a first portion having a first color, a second portion having a second color, the first color being distinguishable from the second color, and the first color and the second color being distinguishable from tissue. The tissue interface can be produced by providing a first part having a first hue; and providing a second part having a second hue. The first hue and the second hue can be distinguishable from each other and a tissue site. The first part can be joined to the second part to form a whole having visually distinguishable sections.

Objectives, advantages, and a preferred mode of making and using the claimed subject matter may be understood best by reference to the accompanying drawings in conjunction with the following detailed description of illustrative embodiments.

### BRIEF DESCRIPTION OF THE DRAWINGS

Figure 1 is a sectional schematic view of an example embodiment of a therapy system that can provide a sealed therapeutic environment in accordance with this specification;
Figure 2 is a perspective view of a tissue interface, illustrating additional details that may be associated with an example embodiment of the therapy system of Figure 1;
Figure 3A is perspective view of a first tissue interface having a first contrasting marker, illustrating additional details that may be associated with a manufacturing process of the tissue interface of Figure 2;
Figure 3B is perspective view of a second tissue interface having a second contrasting marker, illustrating additional details that may be associated with the manufacturing process of the tissue interface of Figure 2;
Figure 4A is perspective view of the first tissue interface having the first contrasting marker, illustrating additional details that may be associated with the manufacturing process of the tissue interface of Figure 2;
Figure 4B is perspective view of the second tissue interface having the second contrasting marker, illustrating additional details that may be associated with the manufacturing process of the tissue interface of Figure 2;
Figure 5A is perspective view of the first tissue interface having the first contrasting marker, illustrating additional details that may be associated with the manufacturing process of the tissue interface of Figure 2;
Figure 5B is perspective view of the second tissue interface having the second contrasting marker, illustrating additional details that may be associated with the manufacturing process of the tissue interface of Figure 2;
Figure 6 is a perspective exploded view of the tissue interface of Figure 2, illustrating additional details that may be associated the manufacturing process of the tissue interface of Figure 2;
Figure 7 is a perspective view of another tissue interface illustrating additional details that may be associated with an example embodiment of the therapy system of Figure 1;
Figure 8 is a perspective color view of the tissue interface of Figure 7; and
Figure 9 is a schematic view of a manufacturing system for example embodiments of the tissue interfaces of Figures 2-8.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

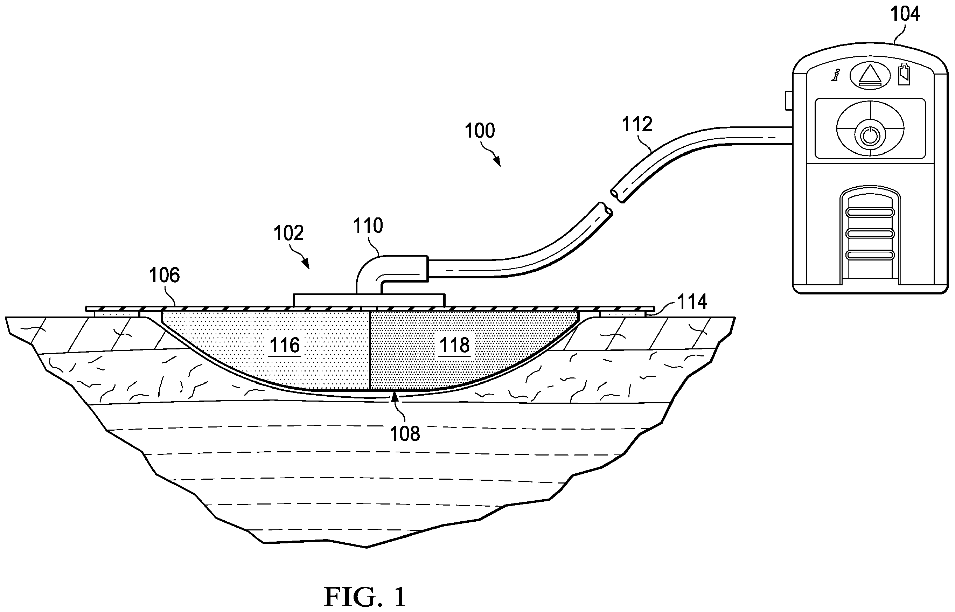

Figure 1 is a sectional schematic view of an example embodiment of a therapy system 100 that can provide a sealed therapeutic environment, negative-pressure therapy, and/or instillation of topical treatment solutions in accordance with this specification. The term "tissue site" in this context broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a negative-pressure supply, and may include or be configured to be coupled to a distribution component, such as a dressing. In general, a distribution component may refer to any complementary or ancillary component configured to be fluidly coupled to a negative-pressure supply in a fluid path between a negative-pressure supply and a tissue site. A distribution component is preferably detachable, and may be disposable, reusable, or recyclable. For example, a dressing 102 may be fluidly coupled to a negative-pressure source 104, as illustrated in Figure 1. A dressing may include a cover, a tissue interface, or both in some embodiments. The dressing 102, for example, may include a cover 106 and a tissue interface 108. A regulator or a controller may also be coupled to the negative-pressure source 104 or a component of the negative-pressure source 104.

In some embodiments, a dressing interface 110 may facilitate coupling the negative-pressure source 104 to the dressing 102. For example, such a dressing interface may be a T.R.A.C.^{®} Pad or Sensa T.R.A.C.^{®} Pad available from KCI of San Antonio, Texas. The therapy system 100 may optionally include a fluid container coupled to the dressing 102 and to the negative-pressure source 104.

The therapy system 100 may also include a source of instillation solution. For example, a solution source may be fluidly coupled to the dressing. The solution source may be fluidly coupled to a positive-pressure source in some embodiments, or may be fluidly coupled to the negative-pressure source 104. A regulator may also be fluidly coupled to the solution source and the dressing 102. In some embodiments, a regulator may also be fluidly coupled to the negative-pressure source 104 through the dressing 102.

Additionally, the therapy system 100 may include sensors to measure operating parameters and provide feedback signals to the controller indicative of the operating parameters. For example, the therapy system 100 may include a pressure sensor, an electric sensor, or both, coupled to the controller. The pressure sensor may also be coupled or configured to be coupled to a distribution component and to the negative-pressure source 104.

Components may be fluidly coupled to each other to provide a path for transferring fluids (i.e., liquid and/or gas) between the components. For example, components may be fluidly coupled through a fluid conductor, such as a tube. A "tube," as used herein, broadly includes a tube, pipe, hose, conduit, or other structure with one or more lumina adapted to convey a fluid between two ends. Typically, a tube is an elongated, cylindrical structure with some flexibility, but the geometry and rigidity may vary. In some embodiments, components may also be coupled by virtue of physical proximity, being integral to a single structure, or being formed from the same piece of material. Moreover, some fluid conductors may be molded into or otherwise integrally combined with other components. Coupling may also include mechanical, thermal, electrical, or chemical coupling (such as a chemical bond) in some contexts. For example, a tube may mechanically and fluidly couple the dressing 102 to a container in some embodiments.

In general, components of the therapy system 100 may be coupled directly or indirectly. For example, the negative-pressure source 104 may be directly coupled to a controller, and may be indirectly coupled to the dressing 102 through a tube 112.

The fluid mechanics of using a negative-pressure source to reduce pressure in another component or location, such as within a sealed therapeutic environment, can be mathematically complex. However, the basic principles of fluid mechanics applicable to negative-pressure therapy and instillation are generally well-known to those skilled in the art, and the process of reducing pressure may be described illustratively herein as "delivering," "distributing," or "generating" negative pressure, for example.

In general, exudates and other fluids flow toward lower pressure along a fluid path. Thus, the term "downstream" typically implies a position in a fluid path relatively closer to a source of negative pressure or further away from a source of positive pressure. Conversely, the term "upstream" implies a position relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications (such as by substituting a positive-pressure source for a negative-pressure source) and this descriptive convention should not be construed as a limiting convention.

"Negative pressure" generally refers to a pressure less than a local ambient pressure, such as the ambient pressure in a local environment external to a sealed therapeutic environment provided by the dressing 102. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. Similarly, references to increases in negative pressure typically refer to a decrease in absolute pressure, while decreases in negative pressure typically refer to an increase in absolute pressure. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -75 mm Hg (-9.9 kPa) and -300 mm Hg (-39.9 kPa).

A negative-pressure supply, such as the negative-pressure source 104, may be a reservoir of air at a negative pressure, or may be a manual or electrically-powered device that can reduce the pressure in a sealed volume, such as a vacuum pump, a suction pump, a wall suction port available at many healthcare facilities, or a micro-pump, for example. A negative-pressure supply may be housed within or used in conjunction with other components, such as sensors, processing units, alarm indicators, memory, databases, software, display devices, or user interfaces that further facilitate therapy. For example, in some embodiments, the negative-pressure source 104 may be combined with the controller and other components into a therapy unit. A negative-pressure supply may also have one or more supply ports configured to facilitate coupling and de-coupling the negative-pressure supply to one or more distribution components.

The tissue interface 108 can be generally adapted to contact a tissue site to act as a wound filler, a device used to fill the negative space of a tissue site. The tissue interface 108 may be partially or fully in contact with the tissue site. If the tissue site is a wound, for example, the tissue interface 108 may partially or completely fill the wound, or may be placed over the wound. The tissue interface 108 may take many forms, and may have many sizes, shapes, or thicknesses depending on a variety of factors, such as the type of treatment being implemented or the nature and size of a tissue site. For example, the size and shape of the tissue interface 108 may be adapted to the contours of deep and irregular shaped tissue sites. Moreover, any or all of the surfaces of the tissue interface 108 may have projections or an uneven, course, or jagged profile that can induce strains and stresses on a tissue site, which can promote granulation at the tissue site.

In some embodiments, the tissue interface 108 may be a manifold. A "manifold" in this context generally includes any substance or structure providing a plurality of pathways adapted to collect or distribute fluid across a tissue site under pressure. For example, a manifold may be adapted to receive negative pressure from a source and distribute negative pressure through multiple apertures across a tissue site, which may have the effect of collecting fluid from across a tissue site and drawing the fluid toward the source. In some embodiments, the fluid path may be reversed or a secondary fluid path may be provided to facilitate delivering fluid across a tissue site.

In some illustrative embodiments, the pathways of a manifold may be interconnected to improve distribution or collection of fluids across a tissue site. In some illustrative embodiments, a manifold may be a porous foam material having interconnected cells or pores. For example, cellular foam, open-cell foam, reticulated foam, porous tissue collections, and other porous material such as gauze or felted mat generally include pores, edges, and/or walls adapted to form interconnected fluid channels. Liquids, gels, and other foams may also include or be cured to include apertures and fluid pathways. In some embodiments, a manifold may additionally or alternatively comprise projections that form interconnected fluid pathways. For example, a manifold may be molded to provide surface projections that define interconnected fluid pathways.

The average pore size of a foam may vary according to needs of a prescribed therapy. For example, the tissue interface 108 may be a foam having pore sizes in a range of about 400 to about 600 microns. The tensile strength of the tissue interface 108 may also vary according to needs of a prescribed therapy. For example, the tensile strength of a foam may be increased for instillation of topical treatment solutions. In one non-limiting example, the tissue interface 108 may be an open-cell, reticulated polyurethane foam such as GranuFoam^{®} dressing or VeraFlo^{®} foam, both available from Kinetic Concepts, Inc. of San Antonio, Texas.

The tissue interface 108 may be either hydrophobic or hydrophilic. In an example in which the tissue interface 108 may be hydrophilic, the tissue interface 108 may also wick fluid away from a tissue site, while continuing to distribute negative pressure to the tissue site. The wicking properties of the tissue interface 108 may draw fluid away from a tissue site by capillary flow or other wicking mechanisms. An example of a hydrophilic foam is a polyvinyl alcohol, open-cell foam such as V.A.C. WhiteFoam^{®} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

The tissue interface 108 may further promote granulation at a tissue site when pressure within the sealed therapeutic environment is reduced. For example, any or all of the surfaces of the tissue interface 108 may have an uneven, coarse, or jagged profile that can induce microstrains and stresses at a tissue site if negative pressure is applied through the tissue interface 108.

In some embodiments, the tissue interface 108 may be constructed from bioresorbable materials. Suitable bioresorbable materials may include, without limitation, a polymeric blend of polylactic acid (PLA) and polyglycolic acid (PGA). The polymeric blend may also include without limitation polycarbonates, polyfumarates, and capralactones. The tissue interface 108 may further serve as a scaffold for new cell-growth, or a scaffold material may be used in conjunction with the tissue interface 108 to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

In some embodiments, the cover 106 may provide a bacterial barrier and protection from physical trauma. The cover 106 may also be constructed from a material that can reduce evaporative losses and provide a fluid seal between two components or two environments, such as between a therapeutic environment and a local external environment. The cover 106 may be, for example, an elastomeric film or membrane that can provide a seal adequate to maintain a negative pressure at a tissue site for a given negative-pressure source. The cover 106 may have a high moisture-vapor transmission rate (MVTR) in some applications. For example, the MVTR may be at least 300 g/m^2 per twenty-four hours in some embodiments. In some example embodiments, the cover 106 may be a polymer drape, such as a polyurethane film, that is permeable to water vapor but impermeable to liquid. Such drapes typically have a thickness in the range of about 25 microns to about 50 microns. For permeable materials, the permeability generally should be low enough that a desired negative pressure may be maintained.

An attachment device 114 may be used to attach the cover 106 to an attachment surface, such as undamaged epidermis, a gasket, or another cover. The attachment device 114 may take many forms. For example, an attachment device may be a medically-acceptable, pressure-sensitive adhesive that extends about a periphery, a portion, or an entire sealing member. In some embodiments, for example, some or all of the cover 106 may be coated with an acrylic adhesive having a coating weight between about 25 to about 65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

A controller may be a microprocessor or computer programmed to operate one or more components of the therapy system 100, such as the negative-pressure source 104. In some embodiments, for example, the controller may be a microcontroller, which generally comprises an integrated circuit containing a processor core and a memory programmed to directly or indirectly control one or more operating parameters of the therapy system 100. Operating parameters may include the power applied to the negative-pressure source 104, the pressure generated by the negative-pressure source 104, or the pressure distributed to the tissue interface 108, for example. The controller is also preferably configured to receive one or more input signals, such as a feedback signal, and programmed to modify one or more operating parameters based on the input signals.

Sensors, such as the pressure sensor or the electric sensor, are generally known in the art as any apparatus operable to detect or measure a physical phenomenon or property, and generally provide a signal indicative of the phenomenon or property that is detected or measured. For example, the pressure sensor and the electric sensor may be configured to measure one or more operating parameters of the therapy system 100. In some embodiments, a pressure sensor may be a transducer configured to measure pressure in a pneumatic pathway and convert the measurement to a signal indicative of the pressure measured. For example, a pressure sensor may be a piezoresistive strain gauge. An electric sensor may optionally measure operating parameters of the negative-pressure source, such as the voltage or current, in some embodiments. Preferably, the signals from a pressure sensor and an electric sensor are suitable as an input signal to the controller, but some signal conditioning may be appropriate in some embodiments. For example, the signal may need to be filtered or amplified before it can be processed by the controller. Typically, the signal is an electrical signal, but may be represented in other forms, such as an optical signal.

A container is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A solution source may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

In operation, the tissue interface 108 may be placed within, over, on, or otherwise proximate to a tissue site. The cover 106 may be placed over the tissue interface 108 and sealed to an attachment surface near the tissue site. For example, the cover 106 may be sealed to undamaged epidermis peripheral to a tissue site with the attachment device 114. Thus, the dressing 102 can provide a sealed therapeutic environment proximate to a tissue site, substantially isolated from the external environment, and the negative-pressure source 104 can reduce the pressure in the sealed therapeutic environment through the dressing interface 110. Negative pressure applied across the tissue site through the tissue interface 108 in the sealed therapeutic environment can induce macrostrain and microstrain in the tissue site, as well as remove exudates and other fluids from the tissue site, which can be collected in the container.

Depending on the type of therapy provided and the duration of therapy, a dressing providing a sealed therapeutic environment over a tissue site can be changed or removed. For example, if negative-pressure therapy has concluded, the dressing can be removed. In another example, negative-pressure therapy encourages a tissue site to heal and close. As the tissue site heals, the tissue site may decrease in size. Consequently, a tissue interface that was selected for the original size of the tissue site, may become too large for the tissue site as the tissue site heals. If negative-pressure therapy has not concluded, the tissue interface may be removed and replaced with a smaller tissue interface.

The tissue interface 108 of the therapy system 100 can assist a user to remove a tissue interface or remove and replace a tissue interface by providing the tissue interface 108 with visually contrasting portions. A tissue interface 108 having visually contrasting portions increases the visibility of the tissue interface 108 in the tissue site. For example, the tissue interface 108 may include contrasting colors, contrasting shapes, or both, causing the tissue interface 108 to appear to be a foreign body. The tissue interface 108 can appear to be a foreign body even if the tissue interface is exposed to copious amounts of exudates or other fluids from the tissue site. In some embodiments, the tissue interface 108 may include portions having a first contrasting marker 116 and different portions having a second contrasting marker 118.

In some embodiments, the first contrasting marker 116 comprises a first color and the second contrasting marker 118 comprises a second color. The first color of the first contrasting marker 116 and the second color of the second contrasting marker 118 are complementary colors. Complementary colors are color pairs that, if mixed, form white or black. If objects formed from two different colors are placed adjacent each other, two colors that are complementary may have a higher visual contrast than colors that are not complementary. Generally, visual contrast increases the visibility of a body. Complementary colors can be determined in a variety of methods in view of a variety of color theories. In different color theories, different colors are complementary pairs. Color theory generally refers to a body of practical guidance for the mixing of colors and the effects of a specific combination of colors. Two example color theories will be explained herein. While other color theories may be used, the two described provide the basic principles by which to understand complementary color pairs. The first color theory is a subtractive color model. The second color theory is an additive color model.

The subtractive color model begins with the concept of white light. Ink, paint, or filters that are applied to a subject absorb wavelengths of the white light, subtracting the hues associated with the absorbed wavelengths. The end result is that the unabsorbed light is reflected back to an observer, providing the color of the object. In the subtractive color model, red, yellow, and blue originally formed the primary colors for the mixing of pigments. A color wheel can be constructed based on the red, yellow, and blue primary colors. A color wheel is an organization of color hues around a circle. The organization illustrates the relationship between the different colors. On the color wheel, red, yellow, and blue occupy positions approximately 120 degrees from each other. Secondary colors, colors formed by mixing of two of the primary colors, are located on the circle between the primary colors from which the secondary color was formed. Where red, yellow, and blue are the primary colors, the secondary colors are orange, green, and purple. Orange is formed by mixing equal parts red and yellow and is located equidistant between red and yellow on the color wheel. Green is formed by mixing equal parts blue and yellow and is located equidistant between blue and yellow on the color wheel. Purple is formed by mixing equal parts blue and red and is located equidistant between blue and red on the color wheel. The secondary colors of green, orange, and purple are also spaced approximately 120 degrees from each other on the color wheel. Colors formed by unequal mixtures of the primary colors of red, yellow, and blue are positioned relative to the primary and secondary colors based on the relative percentages of the primary colors that form the color. For example, color hues having more blue than red are disposed on the color wheel between purple and blue, with the percentage of blue forming the color increasing the closer the color is positioned to blue. Generally, colors which are opposite each other on the color wheel are considered complementary. In the subtractive color model, the complementary pair to red is green, to yellow is purple, and to blue is orange.

The additive color model mixes colors to arrive at an end result. Additive color models add colors of light associated with particular hues, and the resulting combination of colored light produces a resulting color. In the additive color model, the three primary colors are red, green, and blue. As the three primary colors, red, green, and blue will be positioned equidistantly from each other on a color wheel. The secondary colors in the additive color model are yellow, cyan, and magenta. Yellow is formed by mixing equal parts red and green and is located equidistant between red and green on the color wheel. Cyan is formed by mixing equal parts green and blue and is located equidistant between green and blue on the color wheel. Magenta is formed by mixing equal parts blue and red and is located equidistant between blue and red on the color wheel. The secondary colors of yellow, cyan, and magenta are also spaced approximately 120 degrees from each other on the color wheel. Colors formed by unequal mixtures of the primary colors of green, red, and blue are positioned relative to the primary and secondary colors based on the relative percentages of the primary colors that form the color. For example, color hues having more red than blue are disposed on the color wheel between magenta and red, with the percentage of red forming the color increasing the closer the color is positioned to red. The complementary color to red is cyan; the complementary color to green is magenta; and the complementary color to blue is yellow.

Printing and digital representation of color, such as on a computer monitor, generally rely on the additive color model. For many modern printing and digital color representations, the primary colors are cyan, magenta, and yellow, and the complementary pairs are magenta and green, yellow and blue, and cyan and red. In digital reproduction, for example on a computer screen, colors can be defined by a code number identified as an sRGB number, which processors and computer programs use to define the intensity of the color Red, the color Green, and the color Blue for a particular shade. Color can also be represented in a hex triplet, a six-digit, three-byte hexadecimal number used in hypertext markup language ("HTML"), cascading style sheets ("CSS"), scalable vector graphics ("SVG"), and other computer applications to represent colors.

The color red can have a light wavelength between about 620 nanometers (nm) and about 740 nm and has a frequency between about 400 terehertz (THz) and about 480 THz. For red, the sRGB number is 255, 00, 00. In hex triplet, red is represented by the code #FF0000. Traditionally, in the subtractive color model, the complementary color of red is green. In the additive color model, the complementary color of red is cyan. The color cyan has a light wavelength between about 490 nm and about 520 nm and has a frequency between about 575 THz and about 610 THz. In digital representation, the sRGB number is 00, 255, 255, and the hex triplet number is #00FFFF.

The color green can have a light wavelength between about 500 nm and about 575 nm and has a frequency between about 525 THz and about 575 THz. The sRGB number for green is 00, 255, 00, and the hex triplet number is #00FF00. In the additive color model, the complimentary color of green is magenta. The color magenta is a composite color or an extra-spectral color of red and blue and, as a result, is not represented in the visible light spectrum. The sRGB number is 255, 00, 255, and the hex triplet number is #FF00FF.

The color blue can have a light wavelength between about 445 nm and about 500 nm and has a frequency between about 610 THz and about 670 THz. The sRGB number is 00, 00, 255, and the hex triplet representation is #0000FF. Traditionally, in the subtractive color model, the complementary color of blue is orange. The color orange has a light wavelength between about 585 nm and about 620 nm and has a frequency between about 480 THz and about 505 THz. The sRGB number is 255, 128, 00, and the hex triplet number is #FF8000. In the additive color model, the complementary color of blue is yellow. The color yellow has a light wavelength between about 575 nm and about 585 nm and has a frequency between about 512 THz and about 521 THz. The sRGB number is 255, 255, 00, and the hex triplet number is #FFFF00. Traditionally, in the subtractive color model, the complementary color of yellow is purple. The color purple can be considered a composite color or extra-spectral color of red and blue and, as a result, is not represented in the visible light spectrum. In digital representation, the sRGB number is 128, 00, 128, and the hex triplet number is #800080.

The first contrasting markers 116 and the second contrasting markers 118 may typically be primary/complementary color pairs. For example, the first contrasting marker 116 can be red, and the second contrasting marker 118 can be green or cyan. Similarly, if the first contrasting marker 116 is blue, the second contrasting marker 118 can be orange or yellow, and if the first contrasting marker 116 is yellow, the second contrasting marker 118 can be purple or blue. If the first contrasting marker 116 is green, the second contrasting marker 118 can be magenta or red. In some embodiments, the first contrasting marker 116 may be white and the second contrasting marker 118 may be black. In other embodiments, the first contrasting marker 116 may be black and the second contrasting marker 118 may be white. Colors having a hue similar to that of tissue, such as purples or reds, may blend with the tissue of the tissue site and may not be preferred.

The tissue interface 108 includes a first portion having the first contrasting marker 116 and a second portion having the second contrasting marker 118. The first portion having the first contrasting marker 116 and the second portion having the second contrasting marker 118 can form the entirety of the tissue interface 108. In some embodiments, the first portion having the first contrasting marker 116 and the second portion having the second contrasting marker 118 each form approximately half of the tissue interface 108. In other embodiments, the first portion having the first contrasting marker 116 and the second portion having the second contrasting marker 118 may form unequal portions of the tissue interface 108. The first contrasting marker 116 and the second contrasting marker 118 create a visual contrast in the tissue site. The tissue interface 108 being non-monochromatic, creates a visual change as the tissue site is visually scanned from one side to the other, allowing a user to readily identify the tissue interface 108 in the tissue site. If the tissue interface 108 is saturated with fluids from the tissue site, the tissue interface 108 does not appear visually the same as the tissue site is scanned. The complementary colors of the first contrasting marker 116 and the second contrasting marker 118 can cause the portion of the tissue interface 108 having the first contrasting marker 116 to appear visually different from the portion of the tissue interface 108 having the second contrasting marker 118. The juxtaposition of the first contrasting marker 116 and the second contrasting marker 118 can draw the eye of the use, allowing the user to identify the tissue interface 108, even if saturated with fluids from the tissue site.

In some embodiments, fluorescent hues of the complementary color pairs may be used for the first contrasting marker 116 and the second contrasting marker 118. In some embodiments, ornamental patterns may be added to the first contrasting marker 116 and the second contrasting marker 118 to assist in identification of the tissue interface 108. For example, the first contrasting maker 116 and the second and the second contrasting marker 118 can be disposed in portions of the tissue interface 108 so that the portions having the first contrasting marker 116 may form polygonal shapes, including letters, numbers, or other similar features. Similarly, the portions having the second contrasting marker 118 may form polygonal shapes, including letters, numbers, or other similar features.

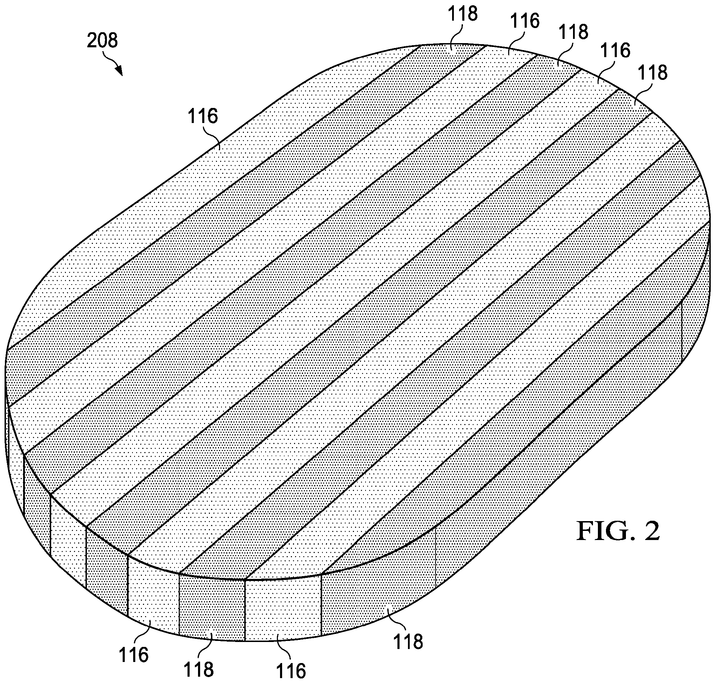

Figure 2 is a perspective view illustrating additional details that may be associated with some example embodiments of another tissue interface 208. The tissue interface 208 may be similar to and operate as described above with respect to the tissue interface 108. The tissue interface 208 can include the first contrasting marker 116 and the second contrasting marker 118 formed into a pattern. For example, the tissue interface 208 can comprise bands or a plurality of strips of the first contrasting markers 116 and a plurality of strips of the second contrasting markers 118. The strips of the first contrasting marker 116 and the strips of the second contrasting marker can be formed into a pattern of repeating parallel strips. The strips of the first contrasting marker 116 and the strips of the second contrasting marker 118 may alternate so that each strip having the first contrasting marker 116 is adjacent to at least one strip having the second contrasting marker 118 and each strip having the second contrasting marker 118 is adjacent to at least one strip having the first contrasting marker 116. Often, each strip having the first contrasting marker 116 may be adjacent to two strips having the second contrasting marker 118, and each strip having the second contrasting marker 118 may be adjacent to two strips having the first contrasting markers 116.

In some embodiments, the tissue interface 208 having the strips of the first contrasting marker 116 and the second contrasting marker 118 can be formed by dying the material of the tissue interface 208. For example, the tissue interface 208 may be formed into its material shape, and a dye may be applied to form the pattern of repeating parallel stripes. In other embodiments, the material of the tissue interface 208 can be dyed in other patterns. For example, the tissue interface 208 can be dyed with the first contrasting marker 116 and the second contrasting marker 118 to have geometric patterns, amorphous patterns, spiral patterns, sunburst patterns, meandering patterns, wave patterns, foam patterns, tiling patterns, crack patterns, rotational patterns, and reflective patterns.

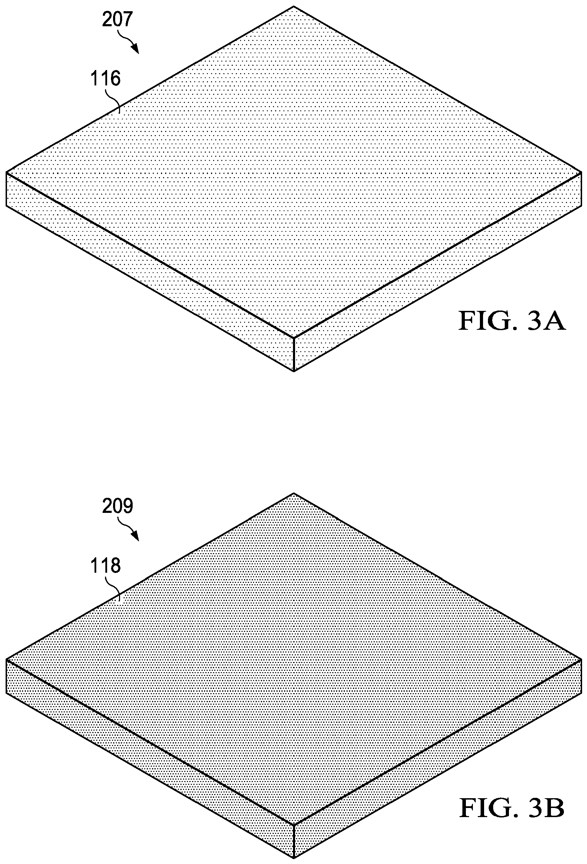

Figure 3A is a perspective view of another tissue interface 207 illustrating additional details that may be associated with some embodiments of a manufacturing process for the tissue interface 208. Figure 3B is a perspective view of another tissue interface 209 illustrating additional details that may be associated with some embodiments of the manufacturing process for the tissue interface 208. The tissue interface 207 of Figure 3A may be similar to and operate as described above with respect to the tissue interface 208. In some embodiments, the tissue interface 207 can be marked with the first contrasting marker 116. The tissue interface 209 of Figure 3B may be similar to and operate as described above with respect to the tissue interface 208. In some embodiments, the tissue interface 209 can be marked with the second contrasting marker 118.

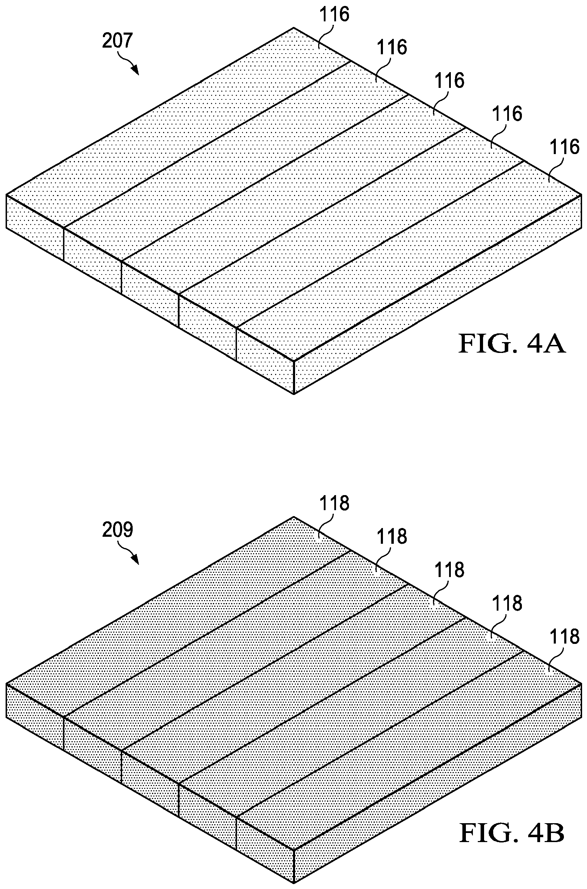

Figure 4A is a perspective view of the tissue interface 207, and Figure 4B is a perspective view of the tissue interface 209, illustrating additional details that may be associated with some embodiments of the manufacturing process for the tissue interface 208. The tissue interface 207 and the tissue interface 209 may be divided into portions. For example, each of the tissue interface 207 and the tissue interface 209 may be cut, torn, melted, or otherwise divided to form smaller portions of each of the tissue interface 207 and the tissue interface 209 having the first contrasting marker 116 and the second contrasting marker 118, respectively.

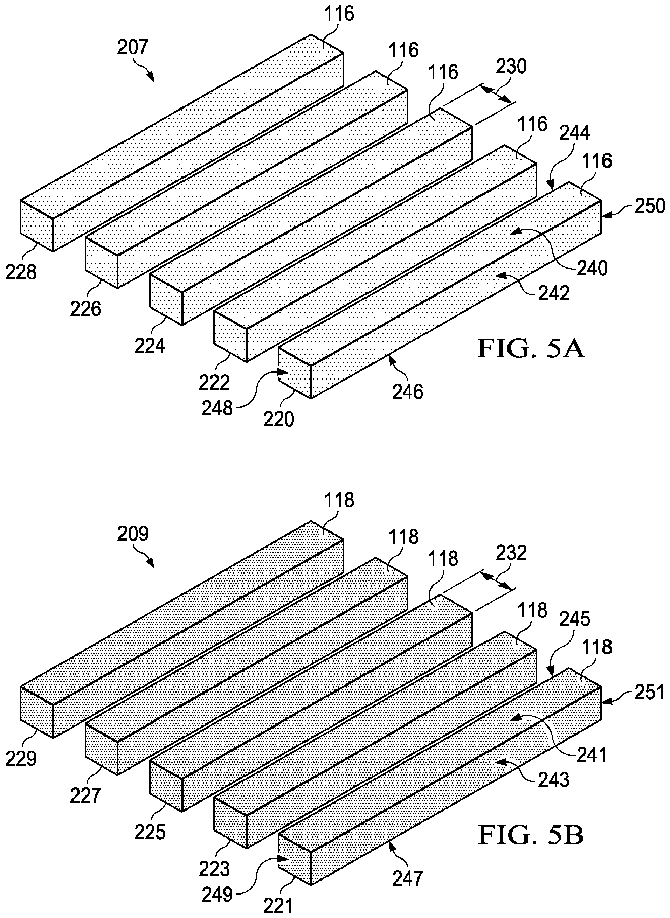

Figure 5A is an exploded perspective view of the tissue interface 207, and Figure 5B is an exploded perspective view of the tissue interface 209, illustrating additional details that may be associated with some embodiments of the manufacturing process for the tissue interface 208. As shown, the divided portions of the tissue interface 207 can be separated to form strips 220, 222, 224, 226, and 228 of the tissue interface 207 having the first contrasting marker 116. Similarly, the divided portions of the tissue interface 209 can be separated to form strips 221, 223, 225, 227, and 229 of the tissue interface 209 having the second contrasting marker 118.

Preferably, the strips 220, 222, 224, 226, and 228 and the strips 221, 223, 225, 227, and 229 are formed having substantially similar sizes and shapes. The strips 220, 222, 224, 226, and 228 may each have a top surface 240, a first surface 242, a second surface 244 opposite the first surface 242, a bottom surface 246 opposite the top surface 240, a first end surface 248, and a second end surface 250 opposite the first end surface 248. The strips 221, 223, 225, 227, and 229 may each a top surface 241, a first surface 243, a second surface 245 opposite the first surface 243, a bottom surface 247 opposite the top surface 241, a first end surface 249, and a second end surface 251 opposite the first end surface 249. In some embodiments, the strips 220, 222, 224, 226, and 228 may each have a width 230, and the strips 221, 223, 225, 227, and 229 may each have a width 232. In some embodiments, the width 230 and the width 232 may be between about 2 mm and about 20 mm. In other embodiments, the width 230 of the strips 220, 222, 224, 226, and 228 and the width 232 of the strips 221, 223, 225, 227, and 229 may be variable from strip to strip.

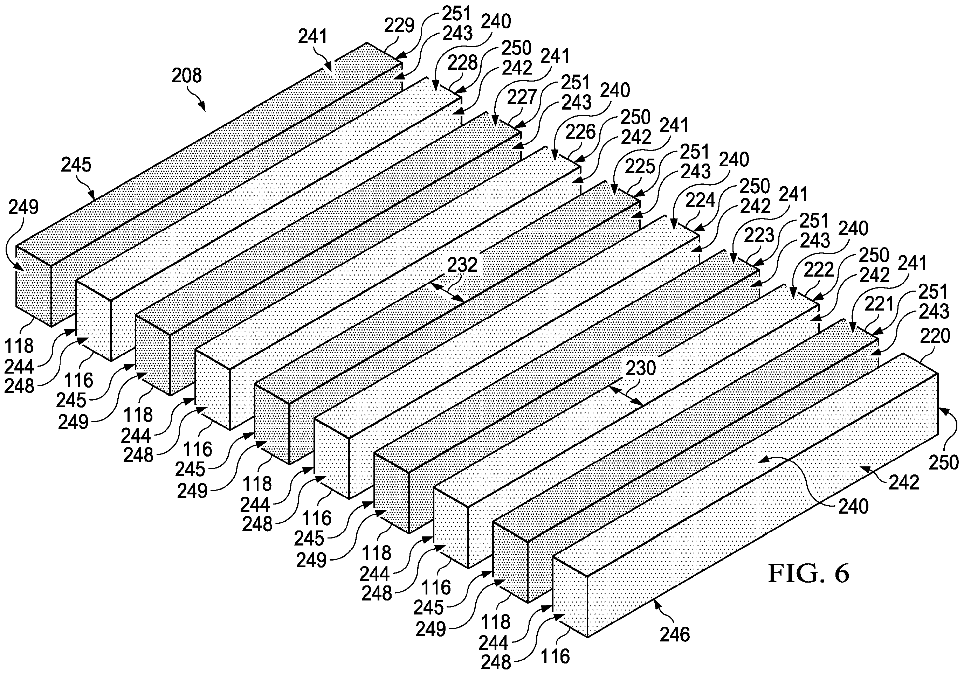

Figure 6 is an exploded perspective view of the tissue interface 208, illustrating additional details that may be associated with some embodiments. The tissue interface 208 can be formed from the strips 220, 222, 224, 226, and 228 of the tissue interface 207 having the first contrasting marker 116 and the strips 221, 223, 225, 227, and 229 of the tissue interface 209 having the second contrasting marker 118. To form the tissue interface 208, the strips 220, 222, 224, 226, and 228 may be coupled to the strips 221, 223, 225, 227, and 229. For example, the strip 220 of the tissue interface 207 can be coupled to the strip 221 of the tissue interface 209. In some embodiments, the strip 220 will be coupled to the strip 221 so that the second surface 244 of the strip 220 contacts and is coupled to the first surface 243 of the strip 221. Similarly, the first surface 242 of the strip 222 can be coupled to the second surface 245 of the strip 221 so that the strip 222 is on an opposite side of the strip 221 from the strip 220. Preferably, the first end surface 248 of the strip 220 can be aligned with the first end surface 249 of the strip 221. Similarly, the second end surface 250 of the strip 220 may be aligned with the second end surface 251 of the strip 221; the top surface 240 can be aligned with the top surface 241; and the bottom surface 246 can be aligned with the bottom surface 247. The strip 223 can be similarly coupled to the strip 222; the strip 224 can be similarly coupled to the strip 223; the strip 225 can be coupled to the strip 224; the strip 226 can be coupled to the strip 225; the strip 227 can be coupled to the strip 226; the strip 228 can be coupled to the strip 227; and the strip 229 can be coupled to the strip 228 to form the tissue interface 208.

Coupling of the strips 220, 222, 224, 226, and 228 and the strips 221, 223, 225, 227, and 229 can be performed by welding, adhesion, bonding, mechanical interlocking, or other coupling mechanisms, such as magnets disposed in the strips 220, 222, 224, 226, and 228 and the strips 221, 223, 225, 227, and 229. For example, the strips 220, 222, 224, 226, and 228 and the strips 221, 223, 225, 227, and 229 can be placed in their respective positions and compressed and heated to fuse contacting surfaces to each other. In some embodiments, the strips 220, 222, 224, 226, and 228 and the strips 221, 223, 225, 227, and 229 can be compressed using a force required to compress an exemplary specimen of the material forming the strips 220, 222, 224, 226, and 228 and the strips 221, 223, 225, 227, and 229 to a height that is about 25% of the specimen's original height. The compression can occur at a temperature between about 100 degrees centigrade and about 200 degrees centigrade, although the temperature can vary depending on the material used for the strips 220, 222, 224, 226, and 228 and the strips 221, 223, 225, 227, and 229. Welding can also include radio-frequency (RF) welding, ultrasonic welding, and hot-plate welding. Adhesion can include application of an adhesive to mating surfaces of the strips 220, 222, 224, 226, and 228 and the strips 221, 223, 225, 227, and 229. Suitable adhesives can include polyurethane adhesives, acrylics, elastomeric adhesives, thermoplastic elastomers, solvent-borne adhesive, water-borne adhesives, and hot-melt adhesive. Mechanical interlocking can include the use of dovetail joints or mortise and tenon joints, hook-and-loop fasteners. After coupling of the strips 220, 222, 224, 226, and 228 and the strips 221, 223, 225, 227, and 229, the tissue interface 208 can be further shaped as needed for distribution and use.

In other embodiments, the strips 220, 222, 224, 226, and 228 and the strips 221, 223, 225, 227, and 229 can be formed into non-linear, curvilinear, or amorphous portions. For example, the strips 220, 222, 224, 226, and 228 and the strips 221, 223, 225, 227, and 229 can be formed into interlocking tiles. Each of the interlocking tiles can be formed such that the surfaces of a strip, for example, the strip 221 can interlock with the surfaces of a corresponding strip 220 and vice versa. In this manner, the tissue interface 208 having non-linear patterns can be constructed by the process described above.

In some embodiments, the tissue interface 208 may be sizeable. For example, at an interface between the strip 220 and the strip 221, the tissue interface 208 may be separated. In some embodiments, fusing of the strips 220, 222, 224, 226, and 228 to the strips 221, 223, 225, 227, and 229 form separation zones in the tissue interface 208. The separation zones comprise areas of the tissue interface 208 where portions of the tissue interface 208 may be preferentially separated from each other. Generally, the fused surfaces of the strips 220, 222, 224, 226, and 228 and the strips 221, 223, 225, 227, and 229 are weaker than the solid portions of each of the strips 220, 222, 224, 226, and 228 and the strips 221, 223, 225, 227, and 229. Consequently, the tissue interface 208 may be separated along the separation zones formed by the interface between each of the strips 220, 222, 224, 226, and 228 and the strips 221, 223, 225, 227, and 229 before breaking of the solid portions of the strips 220, 222, 224, 226, and 228 and the strips 221, 223, 225, 227, and 229.

In other embodiments, the tissue interface 208 may comprise two tissue interfaces 208 formed as above and stacked one on top of the other. The stacked tissue interfaces 208 may be compressed and fused so that the top surfaces 240, 241 of a lower tissue interface 208 may be coupled to the bottom surfaces 246, 247 of an upper tissue interface 208. Similar to the coupling of the first surfaces 242 to the second surfaces 245 and the first surfaces 243 to the second surfaces 244, the coupling of the top surfaces 240, 241 to the bottom surfaces 246, 247 permit the lower tissue interface 208 to be separated from the upper tissue interface 208 to adjust a height of the tissue interface 208. During use, the tissue interfaces 208 may be separable along the contacting surfaces, permitting the tissue interfaces to be sized for particular depths of tissue sites.

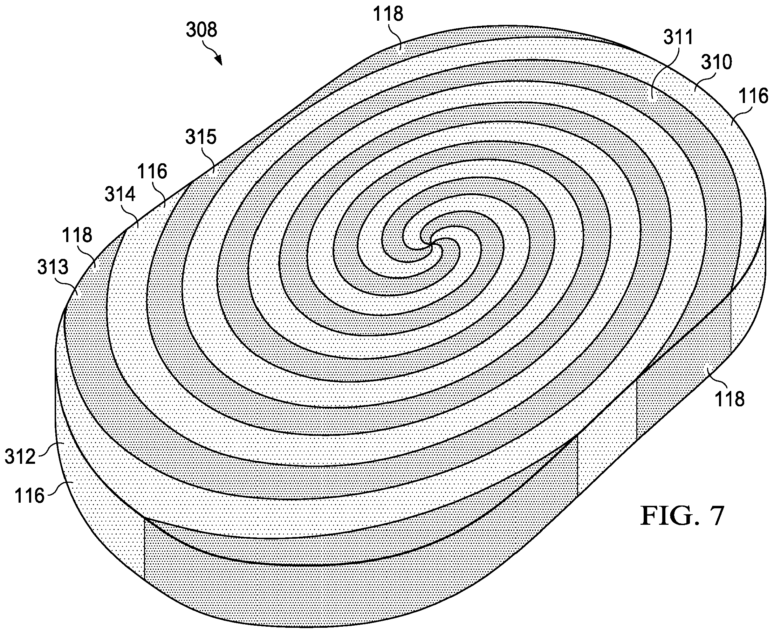

Figure 7 is perspective view of another tissue interface 308 illustrating additional details that may be associated with some embodiments. The tissue interface 308 may be similar to and operate as described above with respect to the tissue interface 108. The tissue interface 308 may include the first contrasting marker 116 and the second contrasting marker 118. In some embodiments, tissue interface 308 includes a plurality of segments. For example, the tissue interface 308 includes segments 310, 312, and 314 having the first contrasting marker 116, and segments 311, 313, and 315 having the second contrasting marker 118.

Each segment 310, 312, and 314 and 311, 313, and 315 may have a top surface, a bottom surface, an interior side surface, and an exterior side surface. The interior side surface and the exterior side surface transcribe a spiral having a fixed center point that continuously increases distance from the fixed center point. The interior side surface may be a side of a segment facing toward the fixed center point, and the exterior side surface may be a side of a segment facing away from the fixed center point. The interior side surface and the exterior side surface can originate from the same fixed center point. The interior side surface may increase at a different rate than the exterior side surface. As a result, each segment 310, 312, and 314 and 311, 313, and 315 increases in thickness from the fixed center point to an outside edge of the tissue interface 308.

The interior surface and the exterior surface of each segment 310, 312, and 314 and 311, 313, and 315 can be correlated so that the exterior surface of a first segment can be placed adjacent to an interior surface of a second segment to form a larger body. For example, the interior surface of the segment 311 can be positioned adjacent to the exterior surface of the segment 310, and the interior surface of the segment 312 can be positioned adjacent to the exterior surface of the segment 311. The segments 310, 312, and 314 and 311, 313, and 315 can coupled to each other to form the tissue interface 308. For example, the exterior surfaces of segments 310, 312, and 314 can be adhered, welded, bonded, or otherwise coupled to the interior surfaces of the segments 311, 313, and 315, respectively. Similarly, the interior surfaces of segments 312 and 314 can be adhered, welded, bonded, or otherwise coupled to the exterior surfaces of segments 311 and 313, respectively. The coupling of segments 310, 312, and 314 to segments 311, 313, and 315 produces the tissue interface 308 having a spiral pattern. The spiral pattern exhibits alternating spirals of the first contrasting marker 116 and the second contrasting marker 118.

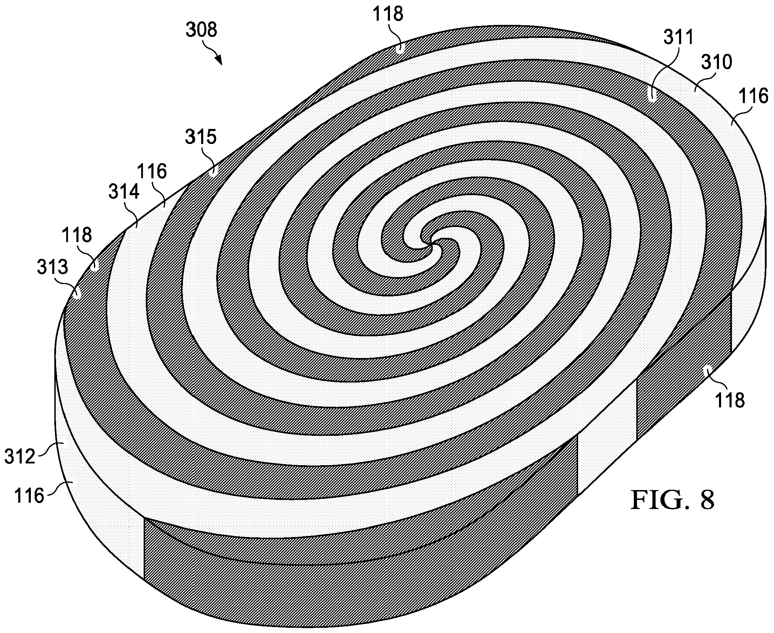

Figure 8 is a perspective view of the tissue interface 308 illustrating additional details of the first contrasting marker 116 and the second contrasting marker 118. The tissue interface 308 includes the segments 310, 312, and 314 coupled to the segments 311, 313, and 315. In the illustrated embodiment, the segments 310, 312, and 314 have the first contrasting marker 116, and the first contrasting marker 116 is yellow. The segments 311, 313, and 315 have the second contrasting marker 118, and the second contrasting marker 118 is blue, a complementary color to yellow. In some embodiments, yellow and blue may be the preferred complementary pairs. For example, the sRGB number for the preferred shade of yellow is 255, 255, 0, and has a light wavelength of about 570 nanometers; the sRGB for the preferred complementary shade of blue is 0, 0, 255 and has a light wavelength of about 475 nanometers.

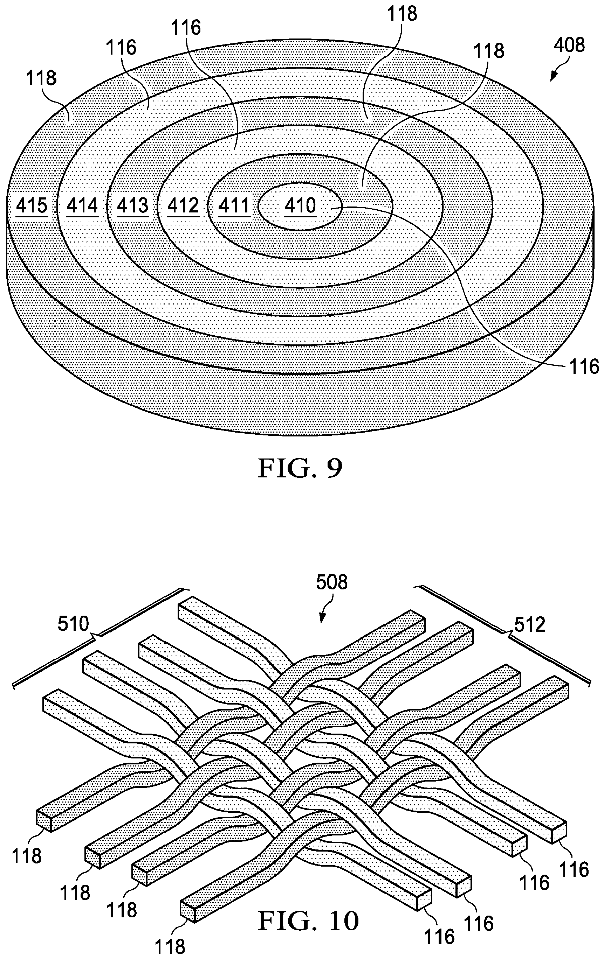

Figure 9 is a perspective view of another tissue interface 408, illustrating additional details that may be associated with some embodiments. The tissue interface 408 includes segments 410, 412, and 414 coupled to segments 411, 413, and 415. In the illustrated embodiment, the segments 410, 2412, and 414 have the first contrasting marker 116. The segments 411, 413, and 415 have the second contrasting marker 118. As shown, the segments 410, 412, and 414 are coupled to the segments 411, 413, and 415 and form concentric rings. The segments 410, 412, and 414 and the segments 411, 413, and 415 are coupled to each other so that the segments 410, 412, and 414 having the first contrasting marker 116 alternate with the segments 411, 413, and 415 having the second contrasting marker. Each successive segment 410, 412, and 414 and segment 411, 413, and 415, has an inner diameter substantially the same as an outer diameter of a previous segment 410, 412, and 414 and segment 411, 413, and 415.

Figure 10 is a perspective view of another tissue interface 508, illustrating additional details that may be associated with some embodiments. In some embodiments, the tissue interface 508 may be a mesh or a woven interface. The mesh or the woven tissue interface 508 may be woven with differently colored threads or fibers having the first contrasting marker 116 and the second contrasting marker 118. For example, the tissue interface 508 may have a first group of threads 510 and a second group of threads 512. The first group of threads 510 may be dyed with the first contrasting marker 116, and the second group of threads 512 may be dyed with the second contrasting marker 118. In some embodiments, the first group of threads 510 may be woven with the second group of threads 512 to create shapes or images of brands or trademarks to permit the first contrasting marker 116 and the second contrasting marker 118 to provide an indication of source. In some embodiments, the first contrasting marker 116 and the second contrasting marker 118 can be printed onto an external surface of the mesh or woven tissue interface 508. In other embodiments, the first group of threads 510 and the second group of threads 512 may be formed into the tissue interface 508 as a non-woven. A non-woven tissue interface 508 can be formed by melt-blown, spun-blown, air-laid, scatter coated, or coupled in another non-woven formation process.

In some embodiments, the tissue interface 108 may have a first color when disposed in a tissue site. After a time period in the tissue site, the tissue interface 108 may have a second color. In some embodiments, the second color may exhibit a pattern. For example, the tissue interface 108 may be black when placed into a tissue site. After approximately 24 hours, the tissue interface 108 may exhibit a pattern similar to the pattern illustrated in Figure 8. In other embodiments, the tissue interface 108 may be white, and after approximately 24 hours may exhibit a pattern similar to the pattern illustrated in Figure 8. In some embodiments, the tissue interface 108 may have a thermochromic pigment disposed in the tissue interface 108. The thermochromic pigment may react with heat, such as body heat; fluid, such as body fluid; or a change in acidity or alkalinity of the environment. For example, the thermochromic pigment may be disposed in the tissue interface 108 in a pattern similar to that illustrated in Figure 8. In response to the heat generated by the tissue site, the thermochromic pigment may react to change color from the first contrasting marker 116 to the second contrasting marker 118. Portions of the tissue interface 108 having the thermochromic pigment can then change from the color, providing a visual contrast in the tissue interface 108.

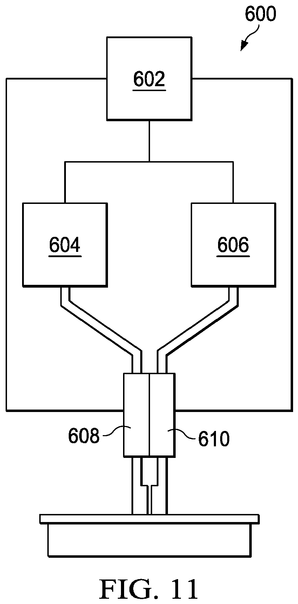

Figure 11 is a schematic depiction of a manufacturing system for producing a tissue interface, such as the tissue interface 108 of Figure 1. The tissue interface 108 may be manufactured in a variety of ways. For example, the tissue interface may be formed from foam, such as GranuFoam ^{®} available from Kinetic Concepts, Inc. of San Antonio, TX. As shown in Figure 11, the tissue interface 108 may be manufactured in an extrusion process. For example, the system 600 may include a controller 602, a first supply 604, a second supply 606, a first extruder 608, and a second extruder 610. The controller 602 may be communicatively coupled to the first supply 604, the second supply 606, the first extruder 608, and the second extruder 610. In some embodiments, the first supply 604 may be in fluid communication with the first extruder 608, and the second supply 606 may be in fluid communication with the second extruder 610.

Ingredients, such as isocyanates, polyols, and pigments may be fed to and mixed in the first supply 604 and the second supply 606. In some embodiments, the first supply 604 and the second supply 606 may include the same or similar ingredients. The first supply 604 and the second supply 606 may be fed different pigments. For example, the first supply 604 may receive a pigment corresponding with the desired color for the first contrasting marker 116, and the second supply 606 may receive pigment corresponding with the desired color for the second contrasting marker 118. The ingredients in the first supply 604 may be fed to the first extruder 608. There, the ingredients can be mixed, a gas can be generated or injected into the ingredients, and the mixed ingredients can expand as they begin to form a polyurethane polymer. The ingredients in the second supply 606 may be fed to the second extruder 610. There, the ingredients can be mixed, a gas can be generated or injected into the ingredients, and the mixed ingredients can expand as they begin to form a polyurethane polymer. The polyurethane polymer in the first extruder 608 and the polyurethane polymer in the second extruder 610 may be extruded onto a surface, such as a platform or assembly line.

In some embodiments, the polyurethane polymer in the first extruder 608 may have the first contrasting marker 116 and the polyurethane polymer in the second extruder 610 may have the second contrasting marker 118. The first extruder 608 and the second extruder 610 may be controlled to provide a stripe pattern similar to the pattern of the tissue interface 208 of Figure 2. In other embodiments, the first extruder 608 and the second extruder 610 may be controlled to provide a swirl pattern similar to the pattern of the tissue interface 308 of Figure 7. The system 600 can produce a tissue interface, such as the tissue interface 108 having the contrasting marker 116 and the contrasting marker 118 extending through the entirety of the tissue interface 108.

In other embodiments, the tissue interface 108 may be batch cast. For example, a container may be filled with a liquid polymer. The liquid polymer may be in a solution form, an emulsion form, or a solid. The liquid polymer may be in the process of foaming. Pigments corresponding to the first contrasting marker 116 and the second contrasting marker 118 may be added to the liquid polymer. The pigments can be added to form patterns, shapes, or other ornamental features as the liquid polymer sets into a foam. In other embodiments, the liquid polymer may foam after the application of a secondary process, such as heating, exposure to ultraviolet light, or similar process. The container may also be filled with the liquid polymer using a co-extrusion process similar to that described and illustrated with respect to the system 600 of Figure 9. In some embodiments, the pigments may be imperfectly mixed in the liquid polymer to provide a jazzed or marbled effect. The liquid polymer may then be cured to form the tissue interface 108. In some embodiments, the tissue interface 108 may be formed from casting or molding polymers. The process can employ extrusion or injection processes similar to those described with respect to the system 600 of Figure 11. In still other embodiments, the tissue interface 108 can be printed from feed stock. For example, a tissue interface 108 may have the first contrasting marker 116 and the second contrasting marker 118 formed on a surface of the tissue interface 108 using a printing process.

The systems, apparatuses, and methods described herein may provide significant advantages. For example, the tissue interfaces described herein provide an adaptation that addresses the long-standing retained material challenge, i.e. material remaining in the tissue site post removal of the tissue interface. The tissue interfaces do not require material reformulations, permitting better identification of the tissue interface in a tissue site without requiring new material, x-ray technology, or magnetic scanners. The contrasting markers augment the visibility of the tissue interface in a tissue site, reducing the likelihood that a tissue interface or portion of a tissue interface may be left in the tissue site. The tissue interfaces described herein also provide additional value to potential users. Specifically, the performance of the tissue interface is increased over similar wound fillers without requiring additional equipment or training for the end user.

While shown in a few illustrative embodiments, a person having ordinary skill in the art will recognize that the systems, apparatuses, and methods described herein are susceptible to various changes and modifications. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing 102, the negative-pressure source 104, or both may be eliminated or separated from other components for manufacture or sale. In other example configurations, the tissue interface 108 may also be manufactured, configured, assembled, or sold independently of other components.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art. Features, elements, and aspects described herein may also be combined or replaced by alternative features serving the same, equivalent, or similar purpose without departing from the scope of the invention defined by the appended claims.

Aspects of the invention are disclosed in the following numbered clauses:
1. A wound filler comprising:
   a first portion having a first color;
   a second portion having a second color, the second color having a contrast to the first color; and
   the first color and the second color having a contrast to red and purple.
2. The wound filler of clause 1, further comprising a third portion having a third color, the third color having a contrast to both the first color and the second color.
3. The wound filler of clause 1 or 2, wherein the first color is white.
4. The wound filler of any of clauses 1-3, wherein the second color is black.
5. The wound filler of any of clauses 1-3, wherein the first color is white and the second color is black.
6. The wound filler of any of clauses 1-5, wherein:
   the first portion has a first pattern; and
   the second portion has a second pattern.
7. The wound filler of clause 6, wherein the first pattern comprises stripes, and the second pattern comprises corresponding stripes.
8. The wound filler of clause 7, wherein the stripes of the first pattern and the second pattern have a width between about 2 mm and about 10 mm.
9. The wound filler of either of clauses 7 or 8, wherein:
   the stripes of the first pattern have a first width;
   the stripes of the second pattern have a second width; and
   the first width is different from the second width.
10. The wound filler of either of clauses 7 or 8, wherein:
   the stripes of the first pattern have a first width;
   the stripes of the second pattern have a second width; and
   the first width is the same as the second width.
11. The wound filler of clause 6, wherein the first pattern comprises first rings, and the second pattern comprises second rings.
12. The wound filler of clause 11, wherein the second rings are concentric with the first rings.
13. The wound filler of clause 6, wherein:
   the first pattern comprises bands having a narrow end proximate to a center of the wound filler and a second end having a wide end proximate to an edge of the wound filler, the wide end having a width greater than the narrow end;
   the second pattern comprises bands having a narrow end proximate to a center of the wound filler and a second end having a wide end proximate to an edge of the wound filler, the wide end having a width greater than the narrow end; and
   wherein the bands of the first pattern alternate with the bands of the second pattern.
14. The wound filler of clause 13, wherein the bands of the first pattern and the bands of the second pattern are curved.
15. The wound filler of either of clauses 13 or 14, wherein the bands of the first pattern and the bands of the second pattern form a sunburst pattern.
16. The wound filler of any of clauses 13, 14, or 15, wherein the bands of the first pattern and the bands of the second pattern form a swirl pattern.
17. The wound filler of any of clauses 1-16, wherein the first portion and the second portion are vertically arranged so that one of the first portion and the second portion is vertically over the other of the second portion and the first portion.
18. The wound filler of any of clauses 1-16, wherein the first portion and the second portion are horizontally arranged so that one of the first portion and the second portion is laterally adjacent the other of the second portion and the first portion.
19. The wound filler of any of clauses 1-18, wherein the first portion and the second portion comprise a mesh.
20. The wound filler of any of clauses 1-19, wherein the first portion comprises fibers of the first color and the second portion comprises fibers of the second color.
21. The wound filler of any of clauses 1-20, wherein the first portion is separable from the second portion.
22. The wound filler of any of clauses 1-21, wherein the first color and the second color are configured to shift in response to exposure to a tissue site.
23. The wound filler of clause 22, wherein the shift comprises changing from a solid color to a pattern.
24. A method of manufacturing a tissue interface:
   providing a first part having a first hue;
   providing a second part having a second hue, wherein the first hue and the second hue are distinguishable from each other and a tissue site; and
   joining the first part to the second part to form a whole having visually distinguishable sections.
25. The method of clause 24, wherein the tissue interface comprises a foam and joining the first part to the second part comprises bonding the first part to the second part.
26. The method of clause 24, wherein the tissue interface comprises a foam and joining the first part to the second part comprises fusing the first part to the second part.
27. The method of any of clauses 24-26, wherein:
   providing the first part comprises:
      feeding first ingredients to a first nozzle,
      mixing the first ingredients in the first nozzle to form a first mixture,
      generating a gas,
      infusing the first mixture with the gas to expand the first mixture, and
      extruding the first mixture with the first nozzle to form a first foam; and
   providing the second part comprises:
      feeding second ingredients to a second nozzle,
      mixing the second ingredients in the second nozzle to form a second mixture,
      generating a gas,
      infusing the second mixture with the gas to expand the second mixture, and
      extruding the second mixture with the second nozzle to form a second foam.
28. The method of clause 27, wherein the first ingredients and the second ingredients comprise isocyanates, polyols, and pigments.
29. The method of any of clauses 24-28, wherein:
   providing the first part comprises feeding first ingredients to a nozzle to form a first mixture;
   providing the second part comprises feeding second ingredients to the nozzle to form a second mixture; and
   the method further comprises:
      co-extruding the first mixture with the nozzle to form a first stripe, and the second mixture with the nozzle to form a second stripe.
30. The method of either of clauses 27 or 29, wherein the first ingredients and the second ingredients comprise isocyanates, polyols, and pigments.
31. The method of any of clauses 24-30, wherein:
   providing the first part comprises:
      filling a first container with a first liquid polymer, and
      heating the first liquid polymer to form a first foam; and
   providing the second part comprises:
      filling a second container with a second liquid polymer, and
      heating the second liquid polymer to form a second foam.
32. The method of any of clauses 24-31, wherein:
   providing the first part comprises:
      extruding a first liquid polymer into a container to form a first stripe;
   providing the second part comprises:
      extruding a second liquid polymer into the container to form a second stripe;
   partially-mixing the first stripe and the second stripe; and
   heating the first liquid polymer and the second liquid polymer.
33. A tissue interface for use with a negative-pressure therapy system, the tissue interface comprising:
   a first portion having a first color formed in a first pattern;
   a second portion having a second color formed in a second pattern;
   the second color having a contrast to the first color; and
   the first color and the second color having a contrast to tissue.
34. The tissue interface of clause 33, further comprising a third portion having a third color formed in a third pattern, the third color having a contrast to both the first color and the second color.
35. The tissue interface of either of clauses 33 or 34, wherein the first color is white.
36. The tissue interface of any of clauses 33-35, wherein the second color is black.
37. The tissue interface of any of clauses 33-35, wherein the first color is white and the second color is black.
38. The tissue interface of any of clauses 33-37, wherein the first color does not include red.
39. The tissue interface of any of clauses 33-38, clauses the first color does not include purple.
40. The tissue interface of any of clauses 33-39, wherein the second color does not include red.
41. The tissue interface of any of clauses 33-40, wherein the second color does not include purple.
42. The tissue interface of any of clauses 33-41, wherein the first color and the second color do not include red and purple.
43. The tissue interface of any of clauses 33-42, wherein the first pattern comprises stripes, and the second pattern comprises corresponding stripes.
44. The tissue interface of clause 43, wherein the stripes of the first pattern and the second pattern have a width between about 2 mm and about 10 mm.
45. The tissue interface of either of clauses 43 or 44, wherein:
   the stripes of the first pattern have a first width;
   the stripes of the second pattern have a second width; and
   the first width is different from the second width.
46. The tissue interface of either of clauses 43 or 44, wherein:
   the stripes of the first pattern have a first width;
   the stripes of the second pattern have a second width; and
   the first width is the same as the second width.
47. The tissue interface of any of clauses 33-46, wherein the first pattern comprises first rings, and the second pattern comprises second rings.
48. The tissue interface of clause 47, wherein the second rings are concentric with the first rings.
49. The tissue interface of any of clauses 33-48, wherein:
   the first pattern comprises bands having a narrow end proximate to a center of the tissue interface and a second end having a wide end proximate to an edge of the tissue interface, the wide end having a width greater than the narrow end;
   the second pattern comprises bands having a narrow end proximate to a center of the tissue interface and a second end having a wide end proximate to an edge of the tissue interface, the wide end having a width greater than the narrow end; and
   wherein the bands of the first pattern alternate with the bands of the second pattern.
50. The tissue interface of clause 49, wherein the bands of the first pattern and the bands of the second pattern are curved.
51. The tissue interface of either of clauses 49 or 50, wherein the bands of the first pattern and the bands of the second pattern form a sunburst pattern.
52. The tissue interface of any of clauses 49-51, wherein the bands of the first pattern and the bands of the second pattern form a swirl pattern.
53. The tissue interface of any of clauses 33-52, wherein the first portion and the second portion are vertically arranged so that one of the first portion and the second portion is vertically over the other of the second portion and the first portion.
54. The tissue interface of any of clauses 33-52, wherein the first portion and the second portion are horizontally arranged so that one of the first portion and the second portion is laterally adjacent the other of the second portion and the first portion.
55. A system for treating a tissue site with negative pressure, the system comprising:
   a manifold configured to be positioned adjacent to the tissue site, the manifold comprising:
      a first portion having a first color;
      a second portion having a second color, the second color having a contrast to the first color; and
      the first color and the second color having a contrast to red and purple;
   a cover configured to be positioned over the manifold to form a sealed space containing the manifold; and
   a negative-pressure source fluidly coupled to the sealed space to draw fluid across the manifold, thereby generating a negative pressure.
56. The system of clause 55, further comprising the manifold having a third portion having a third color, the third color having a contrast to both the first color and the second color.
57. The system of either of clauses 55 or 56, wherein the first color is white.
58. The system of any of clauses 55-57, wherein the second color is black.
59. The system of any of clauses 55-57, wherein the first color is white and the second color is black.
60. The system of any of clauses 55-59, wherein:
   the first portion has a first pattern; and
   the second portion has a second pattern.
61. The system of clause 60, wherein the first pattern comprises stripes, and the second pattern comprises corresponding stripes.
62. The system of clause 61, wherein the stripes of the first pattern and the second pattern have a width between about 2 mm and about 10 mm.
63. The system of clause 61 or 62, wherein:
   the stripes of the first pattern have a first width;
   the stripes of the second pattern have a second width; and
   the first width is different from the second width.
64. The system of either of clauses 61 or 62, wherein:
   the stripes of the first pattern have a first width;
   the stripes of the second pattern have a second width; and
   the first width is the same as the second width.
65. The system of any of clauses 60-64, wherein the first pattern comprises first rings, and the second pattern comprises second rings.
66. The system of clause 65, wherein the second rings are concentric with the first rings.
67. The system of any of clauses 60-66, wherein:
   the first pattern comprises bands having a narrow end proximate to a center of the system and a second end having a wide end proximate to an edge of the system, the wide end having a width greater than the narrow end;
   the second pattern comprises bands having a narrow end proximate to a center of the system and a second end having a wide end proximate to an edge of the system, the wide end having a width greater than the narrow end; and
   wherein the bands of the first pattern alternate with the bands of the second pattern.
68. The system of clause 67, wherein the bands of the first pattern and the bands of the second pattern are curved.
69. The system of either of clauses 67 or 68, wherein the bands of the first pattern and the bands of the second pattern form a sunburst pattern.
70. The system of any of clauses 67-69, wherein the bands of the first pattern and the bands of the second pattern form a swirl pattern.
71. The system of any of clauses 55-70, wherein the first portion and the second portion are vertically arranged so that one of the first portion and the second portion is vertically over the other of the second portion and the first portion.
72. The system of any of clauses 55-70, wherein the first portion and the second portion are horizontally arranged so that one of the first portion and the second portion is laterally adjacent the other of the second portion and the first portion.
73. The system of any of clauses 55-72, wherein the first portion comprises fibers of the first color and the second portion comprises fibers of the second color.
74. The system of any of clauses 55-73, wherein the first portion is separable from the second portion.
75. The system of any of clauses 55-74, wherein the first color and the second color are configured to shift in response to exposure to a tissue site.
76. The system of clause 75, wherein the shift comprises changing from a solid color to a pattern.
77. A tissue interface having a first portion having a first color, a second portion having a second color, the first color distinguishable from the second color, and the first color and the second color distinguishable from tissue, the tissue interfaced produced by a process comprising:
   providing a first part having a first hue;
   providing a second part having a second hue, wherein the first hue and the second hue are distinguishable from each other and a tissue site; and
   joining the first part to the second part to form a whole having visually distinguishable sections.
78. The tissue interface of clause 77, wherein the tissue interface comprises a foam and joining the first part to the second part comprises bonding the first part to the second part.
79. The tissue interface of clause 77, wherein the tissue interface comprises a foam and joining the first part to the second part comprises fusing the first part to the second part.
80. The tissue interface of any of clauses 77-79, wherein:
   providing the first part comprises:
      feeding first ingredients to a first nozzle,
      mixing the first ingredients in the first nozzle to form a first mixture,
      generating a gas,
      infusing the first mixture with the gas to expand the first mixture, and
      extruding the first mixture with the first nozzle to form a first foam; and
   providing the second part comprises:
      feeding second ingredients to a second nozzle,
      mixing the second ingredients in the second nozzle to form a second mixture,
      generating a gas,
      infusing the second mixture with the gas to expand the second mixture, and
      extruding the second mixture with the second nozzle to form a second foam.
81. The tissue interface of clause 80, wherein the first ingredients and the second ingredients comprise isocyanates, polyols, and pigments.
82. The tissue interface of any of clauses 77-81, wherein:
   providing the first part comprises feeding first ingredients to a nozzle to form a first mixture;
   providing the second part comprises feeding second ingredients to the nozzle to form a second mixture; and
   the method further comprises:
      co-extruding the first mixture with the nozzle to form a first stripe, and the second mixture with the nozzle to form a second stripe.
83. The tissue interface of clause 82, wherein the first ingredients and the second ingredients comprise isocyanates, polyols, and pigments.
84. The tissue interface of any of clauses 77-83, wherein:
   providing the first part comprises:
      filling a first container with a first liquid polymer, and
      heating the first liquid polymer to form a first foam; and
   providing the second part comprises:
      filling a second container with a second liquid polymer, and
      heating the second liquid polymer to form a second foam.
85. The tissue interface of any of clauses 77-84, wherein:
   providing the first part comprises:
      extruding a first liquid polymer into a container to form a first stripe;
   providing the second part comprises:
      extruding a second liquid polymer into the container to form a second stripe;
   partially-mixing the first stripe and the second stripe; and
   heating the first liquid polymer and the second liquid polymer.
86. The systems, methods, and apparatuses as described herein.

## Claims

1. A tissue interface for use with a negative-pressure therapy system, the tissue interface comprising:
a first portion having a first color formed in a first pattern;
a second portion having a second color formed in a second pattern;
the second color having a contrast to the first color; and
the first color and the second color having a contrast to tissue.

2. The tissue interface of claim 1, further comprising a third portion having a third color formed in a third pattern, the third color having a contrast to both the first color and the second color.

3. The tissue interface of either of claims 1 or 2, wherein the first color is white.

4. The tissue interface of any of claims 1-3, wherein the second color is black.

5. The tissue interface of any of claims 1-36, wherein the first and/or second color does not include red or purple.

6. The tissue interface of any of claims 1-5, wherein the first pattern comprises stripes, and the second pattern comprises corresponding stripes.

7. The tissue interface of claim 6, wherein the stripes of the first pattern and the second pattern have a width between about 2 mm and about 10 mm.

8. The tissue interface of any of claims 1-7, wherein the first pattern comprises first rings, and the second pattern comprises second rings.

9. The tissue interface of claim 8, wherein the second rings are concentric with the first rings.

10. The tissue interface of any of claims 1-9, wherein:
the first pattern comprises bands having a narrow end proximate to a center of the tissue interface and a second end having a wide end proximate to an edge of the tissue interface, the wide end having a width greater than the narrow end;
the second pattern comprises bands having a narrow end proximate to a center of the tissue interface and a second end having a wide end proximate to an edge of the tissue interface, the wide end having a width greater than the narrow end; and
wherein the bands of the first pattern alternate with the bands of the second pattern.

11. The tissue interface of claim 10, wherein the bands of the first pattern and the bands of the second pattern are curved.

12. The tissue interface of either of claims 10 or 11, wherein the bands of the first pattern and the bands of the second pattern form a sunburst pattern.

13. The tissue interface of any of claims 10-12, wherein the bands of the first pattern and the bands of the second pattern form a swirl pattern.

14. The tissue interface of any of claims 1-13, wherein the first portion and the second portion are vertically arranged so that one of the first portion and the second portion is vertically over the other of the second portion and the first portion.

15. The tissue interface of any of claims 1-13, wherein the first portion and the second portion are horizontally arranged so that one of the first portion and the second portion is laterally adjacent the other of the second portion and the first portion.
